# EUROPÄISCHE PATENTSCHRIFT

(11) **EP 3 194 928 B1**
(45) Veröffentlichungstag und Bekanntmachung des Hinweises auf die Patenterteilung: **03.10.2018**
(21) Anmeldenummer: 15778206.1
(22) Anmeldetag: 20.08.2015
(51) Int. Cl.: G01N 3/58, B27C 3/08, B23Q 17/09, G01N 33/46

(54) **BOHRWIDERSTANDSMESSGERÄT FÜR DIE VERWENDUNG UNTER WASSER**
DRILLING-RESISTANCE MEASURING DEVICE FOR USE UNDERWATER
APPAREIL DE MESURE DE LA RÉSISTANCE AU FORAGE DESTINÉ À UNE UTILISATION SOUS-MARINE

(30) Priorität: 16.09.2014 DE 102014013412
(43) Veröffentlichungstag der Anmeldung: 26.07.2017
(73) Patentinhaber: IML-Instrumenta Mechanik Labor GmbH, 69168 Wiesloch (DE)
(72) Erfinder: HUNGER, Erich, 76133 Karlsruhe (DE); HUNGER, Sebastian, 69181Leimen (DE); HUNGER, Fabian, 69181 Leimen (DE)
(74) Vertreter: Mehl-Mikus, Claudia
(86) Internationale Anmeldenummer: PCT/EP2015/001708
(87) Internationale Veröffentlichungsnummer: WO 2016/041617

(56) Entgegenhaltungen:
- DE-A1- 10 031 334
- DE-A1- 10 031 395
- US-A1- 2006 147 284

## Beschreibung

Die Erfindung betrifft ein Bohrwiderstandsmessgerät zur Materialprüfung mit den Merkmalen des Anspruchs 1, das für die Arbeit und den Einsatz in feuchter Umgebung, insbesondere auch unter Wasser, vorgesehen ist.

Zur Durchführung von Bohrwiderstandsmessungen, mittels derer auf Materialeigenschaften im Inneren von Prüfkörpern geschlossen wird, werden Bohrwiderstandsmessgeräte mit dünnen Bohrwerkzeugen oder Bohrnadeln eingesetzt. Die Bohrnadel wird von einem Antrieb des Bohrwiderstandsmessgeräts bewegt und in den Prüfkörper eingetrieben, wobei beispielsweise die Leistungsaufnahme bzw. der Stromverbrauch des Antriebs als Maß für den Bohrwiderstand des Prüfkörpers beim Eindringen bzw. Herausziehen der Bohrnadel erfasst und beispielsweise mit der Eindringtiefe der Bohrnadel korreliert werden, so dass sich die Materialeigenschaften im Inneren des Prüfkörpers abschätzen lassen.

Bei Messungen in feuchter Umgebung, z. B. in strömendem Regen, oder unter Wasser ist ein Eindringen von Feuchtigkeit bzw. Wasser in das Bohrwiderstandsmessgerät zu verhindern, um Schäden am Antrieb zu vermeiden.

Die DE 100 31 334 A1 beschreibt dazu ein Bohrwiderstandsmessgerät, das eine Austrittsöffnung für die Bohrnadel und Mittel zum Aufbauen eines erhöhten Gasdrucks im Inneren des Bohrwiderstandsmessgeräts aufweist, so dass der im Inneren des Bohrwiderstandsmessgeräts anliegende Druck einem gleichzeitig gemessenen Außendruck entspricht und kein Wasser ins Innere des Bohrwiderstandsmessgeräts eindringen kann. Die Mittel zum Aufbauen eines erhöhten Gas-Innendrucks können eine mit dem Inneren des Bohrwiderstandsmessgeräts gekoppelte Druckgasleitung oder eine angebrachte Gaskartusche sein.

Ausgehend von diesem Stand der Technik ist es Aufgabe der vorliegenden Erfindung, ein Bohrwiderstandsmessgerät bereitzustellen, das unabhängig von einer Druckgasquelle in feuchter Umgebung und unter Wasser funktionsfähig ist.

Diese Aufgabe wird durch ein Bohrwiderstandsmessgerät mit den Merkmalen des Anspruchs 1 gelöst.

Weiterbildungen sind in den Unteransprüchen ausgeführt.
Die weitere Aufgabe, ein Verfahren zur Durchführung einer Bohrwiderstandsmessung in feuchter Umgebung oder unter Wasser ohne Nutzung einer Druckgasquelle anzugeben, wird durch das Verfahren mit den Merkmalen des Anspruchs 11 gelöst.
Ein erfindungsgemäßes Bohrwiderstandsmessgerät zur Materialprüfung in feuchter Umgebung oder unter Wasser weist ein Gehäuse auf, in dem ein Antrieb und ein damit gekoppeltes Bohrfutter angeordnet ist, in dem eine Bohrnadel lösbar aufgenommen oder aufnehmbar ist. Ferner hat das Gehäuse eine von einer Bohrnadelsaustrittsführung umfasste Bohrnadelaustrittsöffnung, durch die sich die in dem Bohrfutter aufgenommene Bohrnadel aus dem Gehäuse erstreckt. Erfindungsgemäß weist das Bohrwiderstandsmessgerät zumindest einen wasserdichten Faltenbalg auf, der im Inneren des Gehäuses zwischen dem Bohrfutter und der Bohrnadelsaustrittsführung um die in dem Bohrfutter aufgenommene oder aufnehmbare Bohrnadel angeordnet ist, wobei Feuchtigkeit oder Wasser durch die Bohrnadelaustrittsöffnung in den Faltenbalg eintreten kann, ohne dass der elektrische Antrieb und Messeinrichtungen im Gehäuse durch Feuchtigkeit in ihrer Funktion beeinträchtigt werden. Erfindungsgemäß wird zugelassen, dass die Bohrnadel im Wasser innerhalb des Faltenbalgs läuft; eine Druckgasquelle ist überflüssig.
Der Faltenbalg ist einenends um das Bohrfutter an einem Antriebsgehäuse innerhalb des Gehäuses und anderenends um die Bohrnadelaustrittsöffnung an der Bohrnadelaustrittsführung oder dem umgebenen Gehäuseabschnitt abdichtend befestigt.

Da Wassereintritt in den Faltenbalg gestattet wird, ist eine kontaktfreie Wirkverbindung zwischen dem Antrieb und dem Bohrfutter eingesetzt, so dass an dieser Stelle der Antrieb gegenüber Feuchtigkeit abgedichtet werden kann. Dazu sind das Bohrfutter und der Antrieb mittels einer Kupplungsvorrichtung verbunden, die eine kontaktfreie Drehmomentübertragung von dem Antrieb auf das Bohrfutter bereitstellt.
Ist - in Abhängigkeit der Länge der Bohrnadel und Art der Faltenbalge - die Anordnung von mehr als einem Faltenbalg zwischen dem Bohrfutter und der Bohrnadelaustrittsführung vorgesehen, so werden die Faltenbalge in Reihe aneinander angeordnet und jeweils über ein Adapterstück miteinander verbunden, das auch der Stabilisierung der Faltenbalge beim Zusammenschieben während des Vorschiebens der Bohrnadel zur Durchführung einer Bohrwiderstandsmessung dient.

Bei der Kupplungsvorrichtung, die eine kontaktfreie Drehmomentübertragung von dem Antrieb auf das Bohrfutter bereitstellt, kann es sich bevorzugt um eine Magnetkupplungsvorrichtung handeln, bei der ein antriebsseitiger Magnetkupplungspartner mit einem bohrfutterseitigen Magnetkupplungspartner in kontaktfreier Wirkverbindung steht, der hierfür eine hohlzylindrische Magnethalterung für ein Aufnahmeelement des Bohrfutters für die Bohrnadel aufweist.

Das Bohrfutter weist ferner einen oder mehrere Klemmkörper und einen Klemmeinsatz auf, der wiederum einen oder mehrere Gegenformkörper aufweist, der oder die den/die Klemmkörper ergänzt/ergänzen, wenn er/sie in einer Ausnehmung an einem zylindrischen Abschnitt des Aufnahmeelements angeordnet ist/sind. Der bzw. die Gegenformkörper erstreckt/erstrecken sich von einer Anschlagscheibe des Klemmeinsatzes weg, die an einer Stirnfläche des zylindrischen Abschnitts anliegt, wenn die Gegenformkörper in der Ausnehmung des Aufnahmeelements aufgenommen sind. Für die zwischen Gegenformkörper und Klemmkörper aufgenommene bzw. aufzunehmende Bohrnadel weist die Anschlagscheibe eine Durchtrittsöffnung auf. Gegenformkörper und Klemmkörper sind daher in Abmessungen und Form zur Anordnung in der Ausnehmung und zur klemmenden Aufnahme eines abgeflachten Endabschnitts der Bohrnadel zwischen den Klemmkörper(n) und den Gegenformkörpern ausgebildet. Durch die plane Fläche ist die lösbar in der Bohrnadelaufnahme aufgenommene Bohrnadel gegen Schlupf gesichert.

Um die durch den abgeflachten Endabschnitt der Bohrnadel bei Rotation entstehende Unwucht auszugleichen bzw. tolerierbar zu halten, so dass die co-zentrische Anordnung der Bohrnadel mit einer Abtriebswelle der Antriebsvorrichtung nicht beeinträchtigt wird, und die Bohrnadel um die gleiche Rotationsachse rotiert wie die Motorabtriebswelle, wird die Kupplungsvorrichtung des Bohrfutters als Magnetkupplung ausgeführt.

Die hohlzylindrische Magnethalterung für das Aufnahmeelement kann beispielsweise durch einen Ringmagneten gebildet werden, der beabstandet und kontaktfrei koaxial um den zylindrischen Abschnitt des Aufnahmeelements angeordnet ist. Bevorzugt kann dabei ein magnetisch beeinflussbarer Distanzring den zylindrischen Abschnitt umgeben, damit die Magnethalterung auf das Aufnahmeelement wirken kann.

Die kontaktfreie Magnetkupplung der Bohrnadelaufnahmevorrichtung mit der Antriebsvorrichtung und die kontaktfreie Anordnung des Aufnahmeelements in dem Bohrfutter gestatten eine gewisse unwuchtige Rotation des Aufnahmeelements, in dem der abgeflachte Endabschnitt aufgenommen ist, wobei jeweils einer der Klemmkörper in dem aus den Gegenformkörpern des Klemmeinsatzes gebildeten Käfig die Klemmung der Bohrnadel übernimmt, je nachdem, wo die Unwucht gerade auftritt.

In einer bevorzugten Ausführungsform ist vorgesehen, dass das Bohrwiderstandsmessgerät eine Ventilvorrichtung aufweist, durch die beim Vorschieben der Bohrnadel, die mit dem Zusammenschieben des Faltenbalgs verbunden ist, zur Durchführung einer Bohrwiderstandsmessung das im Inneren des Faltenbalgs angesammelte Wasser in die Umgebung austreten gelassen wird, wenn ein vorbestimmter Druck erreicht ist. Dazu steht die Ventilvorrichtung mit dem Faltenbalg-Inneren und der Umgebung des Bohrwiderstandsmessgeräts in fluidischer Verbindung, und kann bevorzugt an der Bohrnadelaustrittsführung oder an dem Gehäuse um die Bohrnadelaustrittsführung vorgesehen sein. Diese Ventilvorrichtung öffnet in einer Richtung, um den Wasseraustritt aus dem Inneren des Faltenbalgs in die Umgebung bei Erreichen des vorbestimmten Drucks im Faltenbalg-Inneren zu gestatten, und sperrt in der entgegengesetzten Richtung zumindest bis zu einem vorgegebenen (zweiten) Druck, so dass beim Zurückziehen der Bohrnadel mit dem verbundenen Auseinanderziehen des Faltenbalgs nach Beendigung der Bohrwiderstandsmessung Wasser nicht - bzw. erst ab einem bestimmten, im Inneren des Faltenbalgs durch das Auseinanderziehen erzeugten, von der Rückziehgeschwindigkeit abhängigen Unterdruck - durch die Ventilvorrichtung, sondern nur durch die Bohrnadelaustrittsöffnung ins Innere des Faltenbalgs dringen kann. Ist keine Ventilvorrichtung vorgesehen, so wird das Wasser beim Zusammenschieben des Faltenbalgs aus dem Inneren des Faltenbalgs durch die Bohrnadelaustrittsöffnung gepresst. Das Zusammenschieben des Faltenbalgs kann in beiden Fällen - also mit und ohne Ventilvorrichtung - durch den Antrieb des Bohrwiderstandsmessgeräts unterstützt werden; die erhöhte Leistungsaufnahme im Falle ohne Ventilvorrichtung, die auch von der Einsatztiefe unter Wasser abhängig ist, kann dabei bei der Bohrwiderstandsmessung berücksichtigt werden.

Zur Verbesserung der zentrierten Führung der Bohrnadel zwischen dem Bohrfutter und der Bohrnadelaustrittsführung kann ein erfindungsgemäßes Bohrwiderstandsmessgerät zudem eine teleskopierbare Führungsvorrichtung aus einer Mehrzahl von becherartigen Rohrabschnitten unterschiedlichen Durchmessers aufweisen, in der die Bohrnadel geführt wird. Eine solche teleskopierbare Führungsvorrichtung wird innerhalb des zumindest einen Faltenbalgs angeordnet, so dass sie sich ebenfalls zwischen dem Bohrfutter und der Bohrnadelsaustrittsführung um die Bohrnadel erstreckt. Zudem verhindert eine teleskopierbare Führungsvorrichtung das Abknicken des Faltenbalgs beim Zusammenschieben bzw. beim Vorschieben der Bohrnadel.

Aufgrund der Tatsache, dass Eintritt von Feuchtigkeit/Wasser durch die Bohrnadelaustrittsöffnung gestattet wird, kann in der Bohrnadelaustrittsführung eine Führungshülse ohne besondere Dichtmittel an der Durchtrittsöffnung verwendet werden. Andererseits kann durch die zentrierende Aufnahme in dem Bohrfutter ein Durchmesser der Durchtrittsöffnung an der Führungshülse nahezu entsprechend dem Durchmesser der Bohrnadel gewählt werden, so dass zwischen der Bohrnadel und Wand der Durchtrittsöffnung in der Führungshülse ein minimaler Spalt besteht. Die Spaltbreite sollte höchstens 5 %, vorzugsweise 1 bis 2 % des Bohrnadeldurchmessers betragen. Vorteilhafterweise gelangt durch diesen Spalt beim Zurückziehen der Bohrnadel aus dem Prüfobjekt und in das Gehäuse kein oder kaum Schmutz, etwa abgetragenes Material des Prüfobjekts, ins Geräteinnere - vielmehr werden Materialreste beim Zurückziehen der Bohrnadel durch die Bohrnadelaustrittsführung abgestreift. Ein bevorzugter Bohrnadeldurchmesser liegt bei maximal 3 mm, vorzugsweise bei 1,5 mm, um die gegebenenfalls durch die invasive Bohrwiderstandsmessung erzeugten Schäden möglichst gering zu halten.

Üblicherweise umfassen derartige Bohrwiderstandsmessgeräte auch eine Vorrichtung zur Erfassung des Bohrwiderstands sowie eine Vorrichtung zur Verarbeitung, Speicherung und Ausgabe der erfassten oder verarbeiteten Messwerte. Dabei kann es zur Durchführung von Unterwassermessungen sinnvoll sein, die Vorrichtung zur Erfassung des Bohrwiderstands innerhalb des Gehäuses und die Vorrichtung zur Verarbeitung, Speicherung und Ausgabe der erfassten Bohrwiderstandswerte außerhalb des Gehäuses vorzusehen. Letztere kann dann mit der Vorrichtung zur Erfassung des Bohrwiderstands über Kabel oder drahtlos beispielsweise über eine Funkverbindung verbunden werden oder sein.

Das Bohrwiderstandsmessgerät kann in einer noch weiteren Ausführungsform eine Akku-Einheit zur Versorgung des Antriebs mit elektrischer Energie umfassen, die einen elektrischen Anschluss und einen Rastabschnitt hat. Am Gehäuse des Bohrwiderstandsmessgerätes selbst liegen ein korrespondierender elektrischer Anschluss und ein ebenfalls korrespondierender Gegenrastabschnitt vor. In einer Eingriffsanordnung von Akku-Einheit und Gehäuse steht der Rastabschnitt der Akku-Einheit mit dem Gegenrastabschnitt des Bohrwiderstandsmessgeräts in Eingriff und die elektrischen Anschlüsse der Akku-Einheit und des Bohrwiderstandsmessgeräts kontaktieren sich direkt. Besonders vorteilhaft lässt sich aber der Akku vom Gehäuse entkoppeln, so dass die Antriebseinheit im feuchten oder Staub belasteten Umfeld arbeiten und messen kann, die Strom-, resp. Energieversorgung aber vom Messort beabstandet und im trockenen und geschützten Umfeld platziert werden kann. In dieser entkoppelten Anordnung ist der Rastabschnitt der Akku-Einheit von dem Gegenrastabschnitt des Bohrwiderstandsmessgeräts gelöst und der elektrische Anschluss der Akku-Einheit ist durch ein Kabel mit dem elektrischen Anschluss des Bohrwiderstandsmessgeräts verbunden.

In noch einer weiteren Ausführungsform des Bohrgerätes kann dieses im Antriebsabschnitt einen Kompressor zur Bereitstellung eines Überdrucks im Gehäuse aufweisen. Der Kompressor ist vorteilhaft mit einem Luftfilter verbunden.

In einer bevorzugten Ausführungsform ist ein Luftfilter an der Akku-Einheit angeordnet. Die Akku-Einheit und das Gehäuse weisen Pneumatikanschlüsse auf, die in der Eingriffsanordnung der Akku-Einheit mit dem Gehäuse miteinander verbunden sind. Vorteilhaft kann so aber auch der Luftfilter geschützt positioniert werden, wenn die Akku-Einheit vom Gehäuse entkoppelt vorliegt. Dann sind Akku-Einheit und Kompressor über einen Schlauch miteinander verbunden.

Ein erfindungsgemäßes Verfahren zur Bohrwiderstandsmessung mit einem Bohrwiderstandsmessgerät in feuchter Umgebung oder unter Wasser umfasst folgende Schritte:
- Ansetzen des Bohrwiderstandsmessgeräts in feuchter Umgebung oder unter Wasser an einem zu prüfenden Objekt, dabei
- Eindringen lassen von Feuchtigkeit oder Wasser durch die Bohrnadelaustrittsöffnung in den zumindest einen wasserdichten Faltenbalg, der im Gehäuse des Bohrwiderstandsmessgeräts zwischen der Bohrnadelaustrittsführung und dem Bohrfutter um die Bohrnadel angeordnet ist;
- zur Durchführung der eigentlichen Bohrwiderstandsmessung Vorschieben der Bohrnadel und Einführen in das zu prüfende Objekt, wobei der zumindest eine Faltenbalg zusammengeschoben wird und bei Erreichen eines vorbestimmten Drucks innerhalb des zusammengeschobenen Faltenbalgs die Feuchtigkeit oder das Wasser in die Umgebung des Bohrwiderstandsmessgeräts austreten gelassen werden. Dies kann durch die Bohrnadelaustrittsöffnung, vorzugsweise jedoch durch Öffnen einer mit dem Inneren des zumindest einen Faltenbalgs und der Umgebung in Verbindung stehenden Ventilvorrichtung erfolgen.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Die Figuren sind lediglich schematische Darstellungen einer Ausführungsform der Erfindung.

Dabei zeigen:
- Fig. 1: eine perspektivische Ansicht eines Bohrwiderstandsmessgeräts mit einer über Datenkabel verbundenen Elektronikeinheit,
- Fig. 2: eine schematische Seitenschnittansicht durch einen Teil eines Bohrwiderstandsmessgeräts,
- Fig. 3: eine Seitenschnittansicht durch einen Kopplungsabschnitt zweier Faltenbalge mit einer durch Teleskoprohr geführten Bohrnadel,
- Fig. 4: eine perspektivische Seitenschnittansicht durch eine von drei Faltenbalgen umgebene, mittels dreier Teleskoprohre geführte Bohrnadel mit Bohrnadelaustrittsführung,
- Fig. 5: eine Seitenschnittansicht durch den Anschluss eines Faltenbalgs an die Bohrnadelaustrittsführung ,
- Fig. 6: eine vergrößerte Detaildarstellung eines Abschnitts der Bohrnadelaustrittsführung,
- Fig. 7: eine perspektivische Ansicht des Antriebsabschnitts eines Bohrwiderstandsmessgeräts,
- Fig. 8: eine perspektivische Detailansicht einer Bohrnadelaufnahme am Antriebsgehäuse,
- Fig. 9: eine perspektivische Explosionsansicht der Bohrnadelaufnahme aus Fig. 8,
- Fig. 10: eine perspektivische Detailansicht der Bohrnadelaufnahme aus Fig. 9 mit Bohrnadel,
- Fig. 11: eine perspektivische Ansicht eines Bohrwiderstandsmessgeräts mit entkoppelter, über Leitungen verbundener Akku-Einheit,
- Fig. 12: eine perspektivische Ansicht des Bohrwiderstandsmessgeräts aus Fig. 11 mit angekoppelter Akku-Einheit,
- Fig. 13: eine perspektivische Teilschnittansicht des Bohrwiderstandsmessgeräts aus Fig. 11,
- Fig. 14: eine schematische Darstellung eines Bohrwiderstandsmessgeräts bei Einsatz unter Wasser.

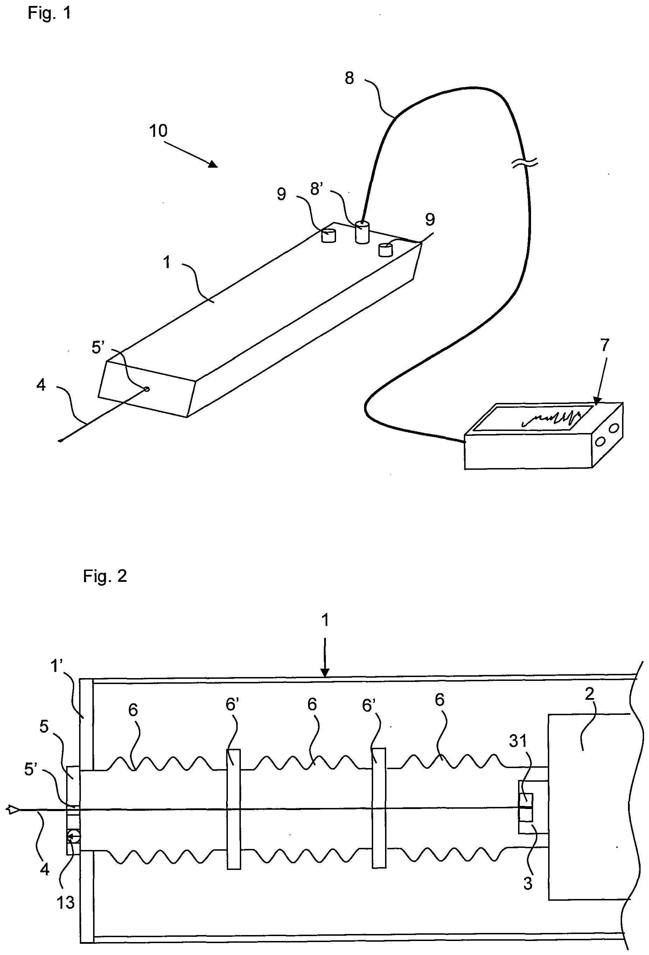

Ein erfindungsgemäßes Bohrwiderstandsmessgerät 10, das zur Bohrwiderstandsmessung unter Wasser oder auch in sehr feuchter Umgebung konzipiert ist, aber selbstverständlich auch für herkömmliche Bohrwiderstandsmessungen an Land eingesetzt werden kann, weist, wie in **Fig. 1** zu sehen ist, ein Gehäuse 1 auf, an dessen Stirnseite eine Bohrnadelaustrittsöffnung 5' vorliegt, durch die sich eine Bohrnadel 4 erstreckt. An dem Gehäuse 1 ist ein Anschluss 8' für eine lange Datenleitung 8 zu sehen, die zu einer Vorrichtung 7 zur Auswertung, Speicherung und/oder Ausgabe der gemessenen Bohrwiderstandwerte führt. Alternativ zu der Leitung 8 kann die Vorrichtung 7 auch über eine drahtlose Kommunikationsverbindung mit einer Vorrichtung zur Erfassung des Bohrwiderstands im Inneren des Gehäuses 1 verbunden sein. Bedienelemente 9 an dem Bohrwiderstandsmessgerät 10 können z.B. zur Einstellung von Bohr- bzw. Messparametern dienen. Die Versorgung des Bohrwiderstandsmessgeräts 10 mit Energie kann mittels Batterien, Akkumulatoren oder über Anschluss an ein Stromnetz erfolgen.

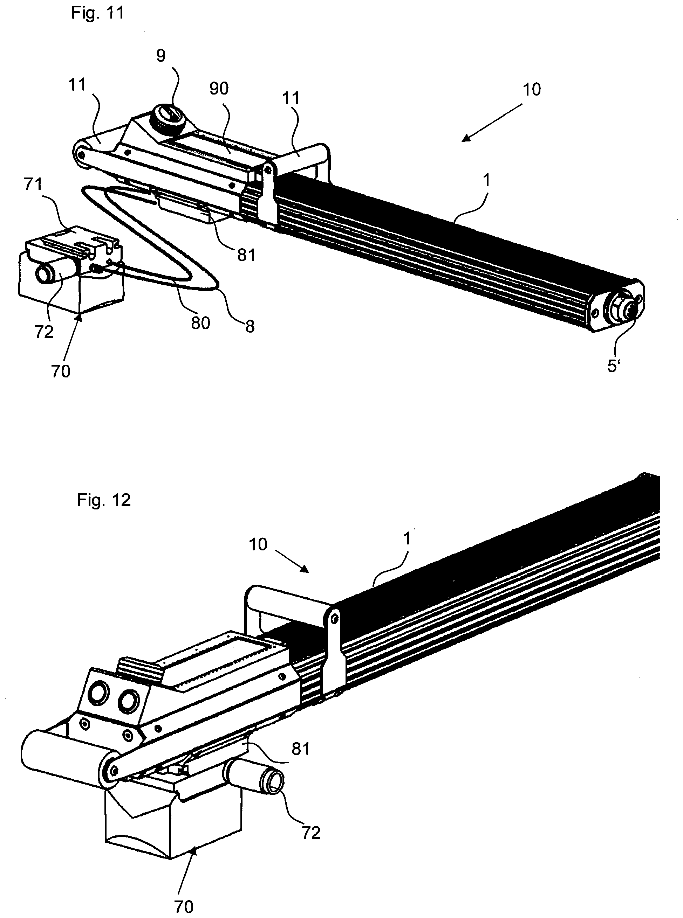

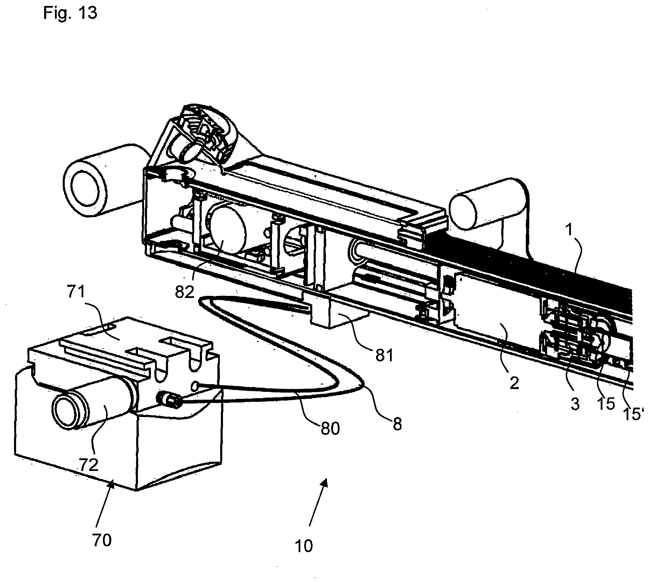

In **Figuren 11 bis 13** ist eine Ausführungsform eines erfindungsgemäßen Bohrwiderstandsmessgeräts 10 dargestellt, das eine entkoppelbare Akkueinheit 70 umfasst. In **Fig. 11** **und** **13** ist die Akku-Einheit 70 von dem Gehäuse 1 des Bohrwiderstandsmessgeräts 10 entkoppelt. Die Stromversorgung des Bohrnadelantriebs im Antriebsgehäuse 2 erfolgt über ein elektrisches Kabel 8 von der Akku-Einheit 70, die somit distanziert vom Einsatzort, beispielsweise außerhalb des Wassers, positioniert sein kann. Die Akku-Einheit 70 weist an ihrer Oberseite einen Rastabschnitt 71 auf, der mit einem korrespondierenden Gegenrastabschnitt 81 am Gehäuse 1 des Bohrwiderstandsmessgeräts 10 in Eingriff gebracht werden kann, wie dies in **Fig. 12** gezeigt ist. Die Rastabschnitte 71, 81 sind so gestaltet, dass der elektrische Anschluss mit dem Einrasten der Akku-Einheit 70 an dem Gehäuse 1 hergestellt wird, die beide entsprechende Anschlüsse (z. B. Stecker und Buchse) aufweisen können. Wenn die Akku-Einheit 70 vom Gehäuse entkoppelt ist, stellt das Kabel 8 die elektrische Verbindung her; entsprechende Anschlüsse liegen an Akku-Einheit 70 und Gehäuse vor.

Die Akku-Einheit 70 und obige EDV-Vorrichtung 7 können separate Vorrichtungen sein, oder aber auch eine gemeinsame Vorrichtung bilden. Zur Datenübertragung und Stromversorgung können daher gegebenenfalls zwei Kabel 8 erforderlich sein.

Andererseits können die mit dem Bohrwiderstandsmessgerät 10 erfassten Daten, wenn sie nicht online über Kabel oder drahtlos an eine beabstandete Vorrichtung 7 übertragen werden, beispielsweise auch auf einer Anzeige 90 des Bohrwiderstandsmessgeräts 10 (siehe **Fig. 11**) angezeigt werden und/oder auf einem integrierten oder einsetzbaren Speichermedium (z. B. Speicherkarte) gespeichert werden, das über eine geeignete Verbindung oder nach Entnahme an eine Auslesevorrichtung angeschlossen werden kann.

Weiter ist in **Fig. 11** **und** **13** ein Schlauch 80 zu sehen, der sich von der Akku-Einheit 70 zu dem Gehäuse 1 erstreckt. Dieser steht mit einem Luftfilter 72 an der Akku-Einheit 70 und einem Kompressor 82 im Gehäuse 1 des Bohrwiderstandsmessgeräts 10 in Verbindung. Hierbei dient die Akku-Einheit 70 auch der Luftversorgung des Antriebsabschnitts des Bohrwiderstandsmessgeräts 10. Der Luftfilter 72 dient der Abtrennung von Feinstaub. Durch die Separierung kann die Akku-Einheit 70 in einem trockenen Umfeld stehen und das Bohrwiderstandsmessgerät 10 kann außer unter Wasser oder in feuchter Umgebung alternativ auch bei hoher Feinstaubbelastung genutzt werden. Der in dem Gehäuse 1 untergebrachte Kompressor 82 erzeugt im Antriebsabschnitt einen Überdruck, so dass dort keine Feinstaubpartikel und/oder Feuchtigkeit eindringen können.

Hierbei wird bemerkt, dass die Ausführungsform mit entkoppelter Akku-Einheit 70 nicht auf die Variante mit Kompressor beschränkt ist. Ein erfindungsgemäßes Bohrwiderstandsmessgerät 10 kann auch lediglich eine entkoppelbare Akku-Einheit 70, wie in den **Figuren 11 bis 13** gezeigt, aufweisen, ohne dass diese Akku-Einheit einen Luftfilter und das Bohrwiderstandsmessgerät einen Kompressor aufweist, die mit einem Schlauch verbunden sind.

Das erfindungsgemäße Bohrwiderstandsmessgerät 10 kann zur Durchführung von Bohrwiderstandsmessungen unter Wasser oder in sehr feuchter Umgebung ohne Druckgasquelle eingesetzt werden, da im Inneren des Gehäuses 1, wie in **Fig.** 2 skizziert ist, eine Faltenbalganordnung, hier aus drei Faltenbalgen 6, zwischen der vereinfacht dargestellten Bohrnadelaustrittsführung 5 und dem Bohrfutter 3 angeordnet ist. Die wasserdicht beispielweise aus Gummi, Kunststoff oder Silikon gefertigten Faltenbalge 6 sind jeweils über stabilisierend wirkende Adapterstücke 6' verbunden und dabei abdichtend an den Adapterstücken 6' sowie endseitig an dem Frontgehäuseabschnitt 1' und an dem Antriebsgehäuse 2 befestigt, so dass das Bohrfutter 3 mit den Klemmkörpern 32 hier innerhalb des Faltenbalgs 6 liegt. Beim Vorschieben der Bohrnadel 4 falten sich die Faltenbalge 6 zusammen, wobei die Adapterstücke 6' ein Einknicken oder Ausweichen der Faltenbalge 6 verhindern, so dass die Bohrnadel 4 in ihrer Bewegung nicht beeinträchtigt wird.

Auf eine spezielle Abdichtung an der Bohrnadeldurchtrittsöffnung 5' wird verzichtet und somit Wassereintritt durch die Bohrnadeldurchtrittsöffnung 5' gestattet, so dass sich der Raum innerhalb der Faltenbalge 6 um die Bohrnadel 4 mit Feuchtigkeit bzw. Wasser füllen kann. Da sowohl die Faltenbalge 6 als auch deren Anbindungen wasserdicht ausgeführt sind und die Kopplung des Bohrfutters 3 mit der Antriebseinheit im Antriebsgehäuse 2 wasserundurchlässig gestaltet ist, wie nachfolgend noch ausgeführt wird, kommen der im Gehäuse 1 untergebrachte elektrische Antrieb und die Vorrichtung zur Erfassung der Bohrwiderstände nicht mit Feuchtigkeit/Wasser in Kontakt, so dass die Funktionsfähigkeit und Messgenauigkeit des Bohrwiderstandsmessgeräts 10 nicht beeinträchtigt wird.

Das Volumen der Faltenbalgvorrichtung wird beim Vorschieben der Bohrnadel 4 zur Durchführung der Bohrwiderstandsmessung komprimiert und dadurch zunehmend Druck in dem innerhalb der Faltenbalgvorrichtung befindlichen Wasservolumen aufgebaut. Damit dieser Druck nicht zu groß wird und den Vorschub der Bohrnadel 4 beeinträchtigt, ist ein Ventil 13 vorgesehen, das ab einem vorbestimmten Druck das Wasser aus dem Inneren der Faltenbalgvorrichtung austreten lässt. In der anderen Richtung, wenn nach Beendigung der Bohrwiderstandsmessung die Bohrnadel zurückgezogen wird, sperrt das Ventil 13 entsprechend einem Rückschlagventil. Wasser bzw. Feuchtigkeit kann nur durch die Bohrnadelaustrittsöffnung 5' eindringen.

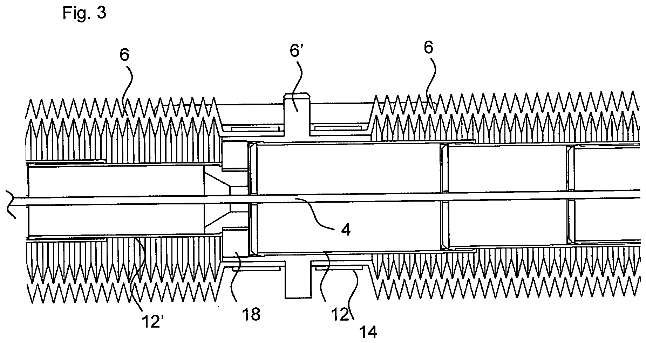

**Fig.** 3 verdeutlicht einen Kopplungsabschnitt zwischen zwei benachbarten Faltenbalgen 6. Das Adapterstück 6' ist hier als Doppelnippel ausgeführt. Die nicht gefalteten Endabschnitte der Faltenbalge 6 sind durch Befestigungsvorrichtungen 14, wie z. B. Schlauch- oder Rohrschellen, an dem Doppelnippel 6' abdichtend befestigt. Eine Hub-Länge der abgedichteten Einheit aus Faltenbalg 6 und Adapterstück 6' ist festgelegt, kann aber so oft wie erforderlich hintereinander montiert werden, mit jeweils einem Adapterstück 6' zwischen benachbarten Faltenbalgen 6, so dass alle Längen - je nach Bohrnadellänge - realisiert werden können.

Weiter ist in **Fig. 3** zu sehen, dass die Bohrnadel 4 innerhalb der Faltenbalgvorrichtung durch Teleskoprohre, von denen jedes aus mehreren becherartigen Rohrabschnitten 12,12' besteht, geführt wird. Beim Vorschieben der Bohrnadel 4 werden die Becher 12,12' jedes Teleskoprohrs ineinander geschoben. Werden, wie vorliegend, mehrere Teleskoprohre eingesetzt, liegt im Bereich der Adapterstücke 6' der Faltenbalgvorrichtung ein Rohrabschnitt 12' mit kleinstem Durchmesser eines ersten Teleskoprohrs am Boden des größten Rohrabschnitts 12 des benachbarten Teleskoprohrs an. Die um die Bohrnadel 4 zentrierte Anordnung von Teleskoprohren und Faltenbalgvorrichtung wird durch eine Lagerung 18 des kleinsten Teleskoprohrabschnitts 12' in dem Adapterstück 6' unterstützt. Das Teleskoprohr ist nicht abgedichtet, so dass innerhalb des Faltenbalgs 6 und damit auch innerhalb der Teleskoprohranordnung um die Bohrnadel 4 Feuchtigkeit bzw. Wasser vorliegen kann, da der Faltenbalg bzw. die Faltenbalge 6 an den Adapterstücken 6' sowie der Bohrnadelaustrittsführung 5 und dem Bohrfutter 4 abgedichtet sind.

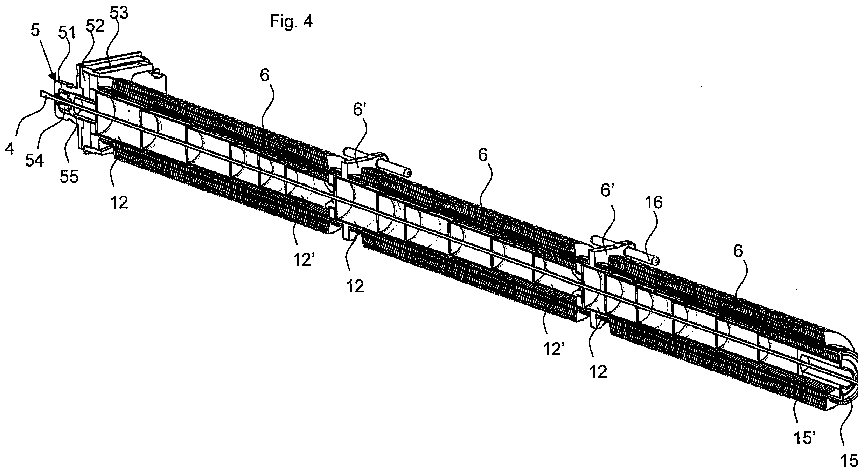

Wie in **Fig. 4** zu sehen ist, kann die Bohrnadel 4 von mehreren Teleskoprohren, quasi von einem aus mehreren Teleskoprohr-Sequenzen zusammengesetztes verlängertes Teleskoprohr, umgeben sein. Die Anzahl der Teleskoprohre und die Anzahl der jeweiligen becherförmigen Rohrabschnitte 12,12' hängt - wie die Anzahl der Faltenbalg-Adapterstück-Einheiten - von der Länge der Bohrnadel 4 ab. Im dargestellten Beispiel korrespondiert die Anzahl der Teleskoprohre mit der Anzahl der Faltenbalge 6, wobei die Adapterstücke 6' zwischen zwei benachbarten Faltenbalgen 6 im Bereich eines Rohrabschnitts 12 mit größtem Durchmesser am Übergang zum Rohrabschnitt 12' mit kleinstem Durchmesser des benachbarten Teleskoprohrs angeordnet sind. Im dargestellten Beispiel weist ein Rohrabschnitt 12 mit größtem Durchmesser zum Arbeitsende des Teleskoprohrs und schließt mit der Bohrnadelaustrittsführung 5 ab, an der auch der entsprechende Faltenbalg 6 abdichtend befestigt ist. Am dazu entgegengesetzten Ende ist der letzte Rohrabschnitt 15' als Andockhülse zusammen mit dem Flanschstück 15, an dem der letzte Faltenbalg 6 befestigt ist, zur abgedichteten Anordnung an dem Antriebsgehäuse (vgl. **Fig. 2**) ausgebildet, während die Bohrnadel 4 von dem Bohrfutter aufgenommen wird (nicht in **Fig. 4** dargestellt). Die Befestigung des letzten Faltenbalgs 6 an dem Flanschstück 15 kann wie an den Adapterstücken durch eine Befestigungsvorrichtung wie eine Schlauchschelle an dem nicht gefalteten Endabschnitt des Faltenbalgs 6 erfolgen.

Grundsätzlich ist es aber auch möglich, die Anordnung der becherartigen Rohrabschnitte 12,12' in Bezug auf die Rohrabschnitt-Durchmesser umgekehrt zu wählen, d. h. ein Becher mit größtem Durchmesser liegt am Andockende und ein Becher mit kleinstem Durchmesser am Arbeitsende vor.

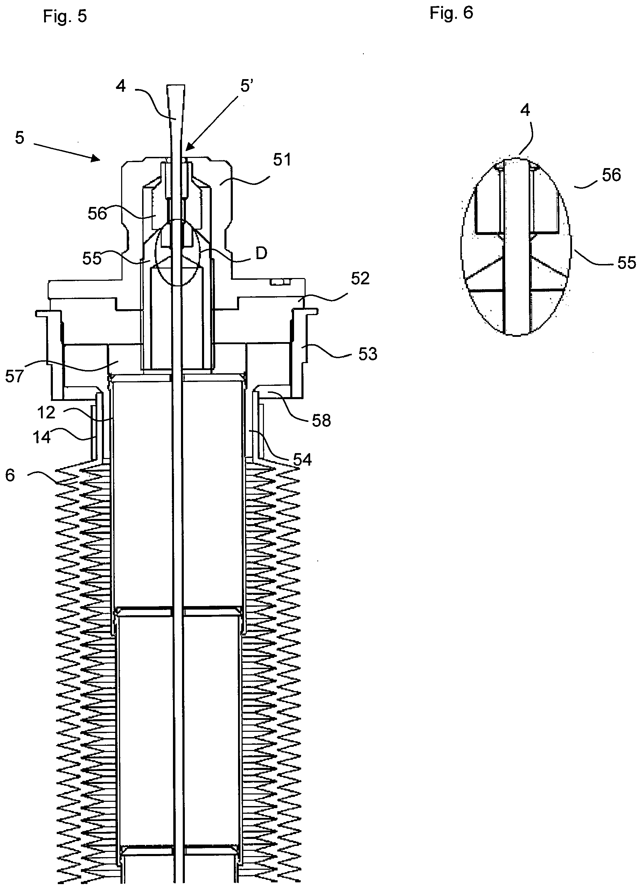

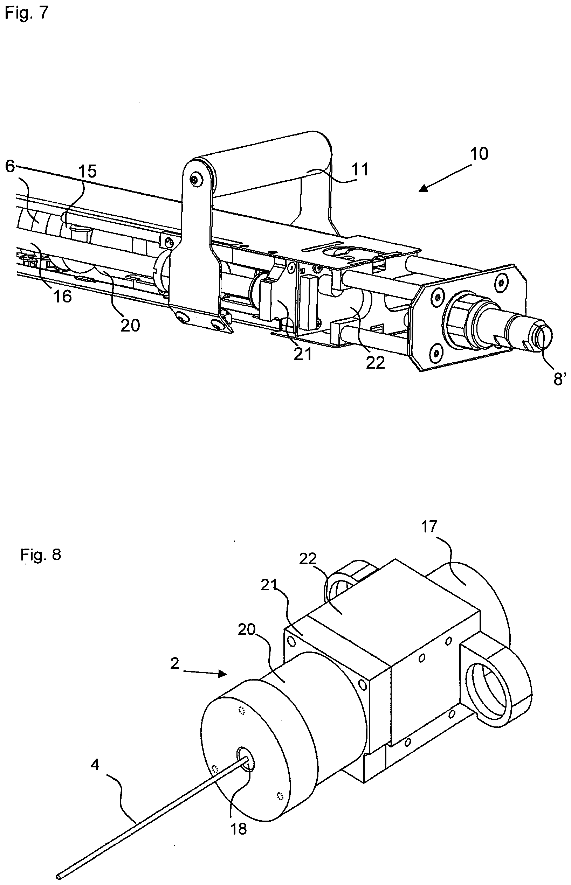

Der Aufbau der Bohrnadelaustrittsführung 5 ist in **Fig. 4** und deutlicher in **Fig. 5** zu sehen. Ein Detail D der Bohrnadelaustrittsführung 5, das eine Stelle zeigt, an der der Durchmesser der Bohrnadel 4 nahezu gleich dem Durchmesser einer Bohrnadeldurchtrittsöffnung in der inneren Führungshülse 55 ist, ist in **Fig. 6** dargestellt. Der Durchmesser der Bohrnadel 4 und der Durchmesser der geringfügig größeren Bohrnadeldurchtrittsöffnung weisen nur wenig Spiel auf: Ein derartig geringer Toleranzbereich lässt sich nur aufgrund der zentrierten Führung der Bohrnadel 4 im Bohrfutter 3 (vgl. **Fig. 8 bis 10**) und damit weitgehend ohne Dichtmaßnahmen an dieser Stelle realisieren.

Die Bohrnadelaustrittsführung 5 setzt sich hier aus einer äußeren Führungshülse 51 mit einer eingeschraubten inneren Führungshülse 55 und einer Überbrückungshülse 56 zusammen, die sich in der äußeren Führungshülse 51 zwischen der inneren Führungshülse 55 und der Bohrnadelaustrittsöffnung 5' erstreckt. Auf die innere Führungshülse 55 sind hintereinander ein Ringflansch 52, der an der äußeren Führungshülse 51 anliegt, und ein Teleskopanschlag 57, der an dem Ringflansch 52 anliegt, aufgeschraubt. Eine Teleskopaufnahmehülse 54 umgibt nicht nur teilweise den letzten Rohrabschnitt 12, sondern auch den Teleskopanschlag 57 und liegt an dem Ringflansch 52 an. Auf dem den Rohrabschnitt 12 umgebenden zylindrischen Abschnitt der Teleskopaufnahmehülse 54 ist der nicht gefaltete Abschnitt des entsprechenden Faltenbalgs 6 mit einer entsprechenden Befestigungsvorrichtung 14 wasserdicht befestigt. Die Teleskopaufnahmehülse 54 wird mittels eines Schraubrings 53 und einer Scheibe 58 an dem Ringflansch 52 befestigt.

**Fig. 7** zeigt den von der Bohrnadelaustrittsöffnung 5' abgewandten Abschnitt des Bohrwiderstandsmessgeräts 10, in dem der Andockflansch 15 an dem Bohrfutter anliegt, und in dem der Antrieb für die Bohrnadel untergebracht ist, der mit Mess- und Steuerungsvorrichtungen sowie dem Anschluss 8' verbunden ist. Die Führungs- bzw. Stabilisierungsstange 16, die abschnittsweise auch an den Adapterstücken 6' in **Fig. 4** zu sehen sind, dient der geradlinigen Führung der Faltenbalge 6 und der Teleskoprohre, wenn diese beim Vorschub der Bohrnadel 4 zusammengedrückt bzw. beim Zurückziehen der Nadel auseinandergezogen werden. Gegebenenfalls kann diese Hin- und Herbewegung auch aktiv mittels der Führungs-/Stabilisierungsstange 16 unterstützt werden, so dass die Teleskoprohre und die Faltenbalge 6 aktiv synchron mit der Bohrnadel 4 bewegt werden.

Im Folgenden werden Details der Bohrnadelaufnahme am Antriebsgehäuse 2 gemäß einer Ausführungsform der Bohrnadelaufnahme in Bezug zu **Fig. 8 bis 10** beschrieben. Der Übersichtlichkeit wegen ist in diesen Figuren, die sich auf das Detail der Bohrnadelaufnahme beziehen, keine Faltenbalgvorrichtung dargestellt.

Auf den Offenbarungsgehalt der Deutschen Patentanmeldung DE 10 2013 015 131 der Anmelderin wird hiermit vollumfänglich Bezug genommen; der Gegenstand der Anmeldung DE 10 2013 015 131 wird damit in diese Anmeldung inkorporiert.

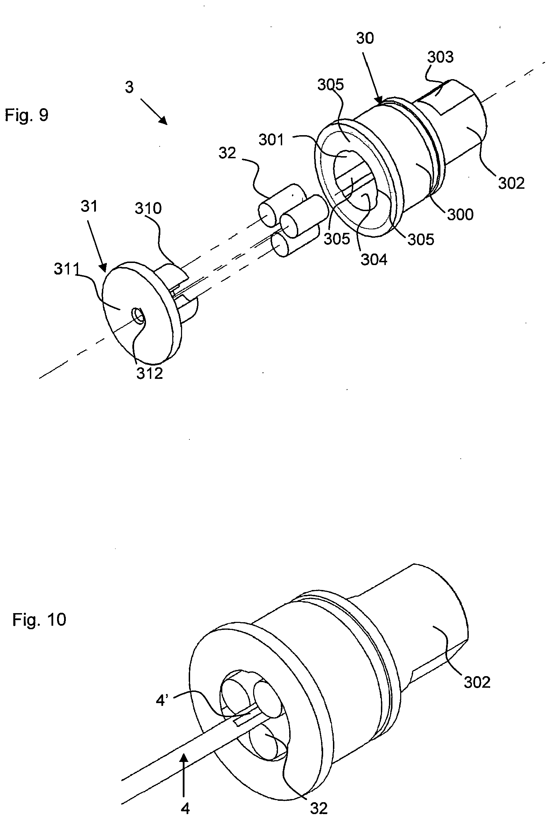

Die in **Fig. 8** gezeigte Bohrnadelaufnahme umfasst in dem Gehäuse 22 eine mit dem Motor 17 verbundene Motorabtriebswelle, die zur kontaktfreien Drehmomentübertragung auf die in einem Aufnahmeelement 30 des Bohrfutters 3 aufgenommene Bohrnadel 4 über eine Magnetkupplung (nicht dargestellt) mit dem Aufnahmeelement 30 (vgl. **Fig. 9**) gekoppelt ist. Durch die kontaktfreie Wirkverbindung zwischen Bohrfutter und Antrieb ist eine wasserdichte Abdichtung an dieser Stelle einfach zu bewerkstelligen.

In dem Aufnahmeelement 30 ist der abgeflachten Endabschnitt 4' der Bohrnadel 4 (vgl. **Fig. 10**) durch die Klemmkörper 32 und den Klemmeinsatz 31 geklemmt. Das den antriebsseitigen Kupplungspartner umgebende Gehäusesegment 21 ist mit dem Motorgehäuse 22 beispielsweise mittels Schrauben oder Stiften verbunden.

Die Bohrnadel 4 ist durch den abgeflachte Endabschnitt 4' in der Bohrnadelaufnahme gegen Schlupf gesichert, jedoch verursacht dieser abgeflachte Endabschnitt 4' eine gewisse Unwucht bei Rotation der Bohrnadel 4. Um dennoch eine zentrische Anordnung der Bohrnadel 4 zu gewährleisten, ist nicht nur die kontaktfreie Magnetkupplung der Bohrnadelaufnahme mit der Antriebsvorrichtung, sondern auch eine kontaktfreie Anordnung des Aufnahmeelements 30 in dem Bohrfutter 3 vorgesehen. Ferner wird die Bohrnadel 4 durch eine Führungshülse 18, die in der Durchtrittsöffnung der Frontabdeckung 2 gelagert ist, axial zentriert und verläuft damit co-zentrisch mit der Motorabtriebswelle und der Bohrnadelaustrittsöffnung 5', unabhängig von den an dem Aufnahmeelement 30 auftretenden exzentrischen Versätzen infolge der Unwucht.

Eine derartige Bohrnadel 4 neigt weniger zu Nadelbrüchen, wobei die Querschnittsabmessung der Bohrnadel 4 rechtwinklig zu der Abflachung des Endabschnitts 4' höchstens 5 %, bevorzugt 3 % kleiner als der Durchmesser der Bohrnadel 4 ist.

Die in **Fig. 8** gezeigte Frontabdeckung 2 kann beispielsweise an der umfänglichen Innenwand ein Gewinde aufweisen, mit dem sie mit dem Gehäuseabschnitt 20, der dann ein entsprechendes Außengewinde aufweist, verschraubt werden kann. Alternativ kann die Frontabdeckung 2 auch unlösbar mit dem Gehäuseabschnitt 20 verbunden, beispielsweise verklebt sein. Der Gehäuseabschnitt 20 ist lösbar mit dem Gehäusesegment 21 verbunden. Für diese Verbindung kann beispielsweise ein Gewinde an einem innenumfänglichen Abschnitt des Gehäuseabschnitts 20 und an einem Außenumfang eines entsprechenden Abschnitts des Gehäusesegments 21 vorgesehen sein. Selbstverständlich sind auch andere Ausführungen der Gehäusekomponenten denkbar.

Zur Verstellung der axialen Distanz zwischen den nicht dargestellten magnetischen Kupplungspartnern kann einfach die Verschraubungstiefe zwischen Gehäuseabschnitt 20 und Gehäusesegment 21 variiert werden, was händisch oder automatisiert mit entsprechenden Stellmitteln erfolgen kann, oder es kann beispielsweise auch ein Gehäuseabschnitt 20 mit einer anderen Länge eingesetzt werden. Weitere Alternativen zur Einstellung der axialen Distanz zwischen den Kupplungspartnern liegen in einer axial verstellbaren Verbindung eines oder beider Kupplungspartner an dem Aufnahmeelement 30 und/oder der Abtriebswelle.

So kann auch bei Wechsel des Bohrfutters 3, wofür der mit der Frontabdeckung 2 verbundene Gehäuseabschnitt 20 von dem Gehäusesegment 21 gelöst wird, durch Einstellung des Abstands der beiden Kupplungspartner das gleiche Übertragungsdrehmoment beibehalten werden.

**Fig. 9 und 10** zeigen weitere Details der Bohrnadelaufnahme. Dort ist das Aufnahmeelement 30 mit dem zylindrischen Abschnitt 300, der die Ausnehmung 301 aufweist, und mit dem Zapfenabschnitt 302 gezeigt, der mit dem einem Magnetkupplungspartner verbunden wird. Der Zapfenabschnitt 302 weist zum verbesserten Eingriff eine Abflachung 303 auf.

Die Wand der Ausnehmung 301 ist entsprechend den Gegenformkörpern 310 des Klemmeinsatzes 31, die die drei Klemmkörper 32 ergänzen, in drei Segmente 304 unterteilt, die durch nach innen weisende Anschläge 305 begrenzt werden. Die Anschlagscheibe 311, mit der der Klemmeinsatz 31 an der Stirnfläche des zylindrischen Abschnitts 300 des Aufnahmeelements 30 zur Anlage kommt, weist eine zentrische Durchtrittsöffnung 312 für die Bohrnadel 4 auf, die mit ihrem abgeflachten Endabschnitt 4' zwischen den Klemmkörpern 32 und den Gegenformkörpern 310 in der Ausnehmung 301 geklemmt wird.

Die Bohrnadel 4 ist mit dem abgeflachten Endabschnitt 4' in der Ausnehmung 301 des Aufnahmeelements 30 zwischen den Klemmkörpern 32 und den Gegenformkörpern 310 aufgenommen. Die infolge des abgeflachten Endabschnitts 4' bei Rotation entstehende Unwucht wird ausgeglichen, indem jeweils einer der Klemmkörper 32 in dem aus den Gegenformkörpern 310 des Klemmeinsatzes 31 gebildeten Käfig die Klemmung der Bohrnadel 4 übernimmt, je nach dem, wo die Unwucht gerade auftritt.

Die Wandsegmente 304 der Ausnehmung 301 weichen in ihrer Gestaltung von einem zentrischen Kreisprofil ab, wobei jedes Wandsegment 304 einem Abschnitt einer Spirallinie um die Bohrnadel 4 entspricht, sodass die Anschläge 305 jeweils den Übergang zwischen einem proximalen und distalen Segmentabschnitt benachbarter Segmente 304 bilden. Werden die Klemmkörper 32 in die distalen Segmentabschnitte bewegt, wird deren Abstand zu der Bohrnadel 4 vergrößert und der Klemmeingriff gelöst. Umgekehrt wird die Bohrnadel 4 zwischen den Klemmkörpern 32 geklemmt, wenn diese in Richtung proximaler Segmentabschnitte bewegt werden.

Die in den **Fig. 8 bis 10** gezeigte Bohrnadelaufnahme ist beispielhaft zu verstehen, der Schutzumfang eines erfindungsgemäßen Bohrwiderstandsmessgeräts ist jedoch nicht darauf beschränkt. So sind andere Bohrfutterausführungen, Gehäuselösungen und Kupplungsvarianten denkbar, ohne den Schutzumfang der Erfindung zu verlassen.

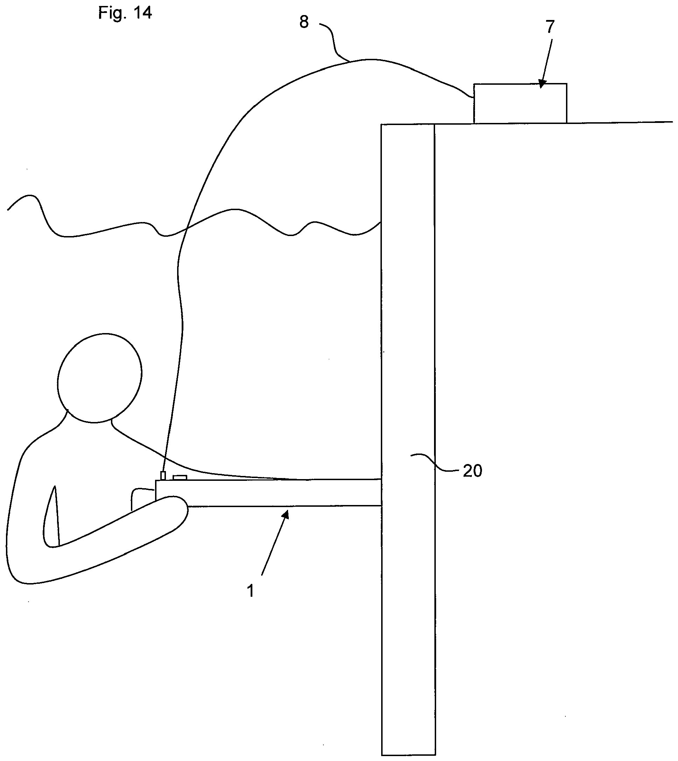

**Fig. 14** zeigt ein erfindungsgemäßes Bohrwiderstandsmessgerät im Einsatz. Ein Taucher führt die Messung an einem Prüfkörper 20 unterhalb der Wasserlinie durch. Messdaten werden über das Verbindungskabel 8 zu der Vorrichtung 7 zur Auswertung, Speicherung und Ausgabe übertragen, die außerhalb des Wassers platziert ist. Anders als dargestellt kommt natürlich auch eine drahtlose Übertragung der Messdaten zu der Vorrichtung 7 in Frage.

Ein erfindungsgemäßes Bohrwiderstandsmessgerät, mit dem in erster Linie Bohrwiderstandsmessungen anhand der Leistungsdaten des Motors aufgenommen werden, kann durchaus gemäß der DE 10 2009 013 069 A1/B4 der Anmelderin weitergebildet sein, die hiermit vollumfänglich durch Bezugnahme umfasst wird. Dabei wird die Beschaffenheit von säulenförmigen oder zylindrischen Abschnitten von Prüfkörpern untersucht, indem die Bohrnadel mit Hilfe eines Bohrgeräts in einem näherungsweise rechten Winkel zu einer Längsachse des Abschnitts des Körpers, der untersucht werden soll, diskontinuierlich eingeführt wird, wobei zum Einen während des Einführens eine Bohrwiderstandsmessung ausgeführt wird und zum Anderen die Aufnahme eines Impulstomogramms durch das diskontinuierliche Einführen ermöglicht wird, bei dem die so erzeugten Impulse von Sensoren erfasst werden, die an dem Umfang des Prüfkörpers verteilt sind. Die Ausbreitungsgeschwindigkeit der Impulswellen hängt im Wesentlichen von der Dichte und Härte des Prüfkörpermaterials auf dem Weg von der Quelle zu den verschiedenen Sensoren ab. So wird die Bohrwiderstandsmessung durch ein Impulstomogramm ergänzt, wodurch mehr Informationen bezüglich des Inneren des Prüfkörpers erhalten werden können bzw. eine Abbildung der Beschaffenheit des Kernmaterials von hoher Präzision geschaffen wird.

**Bezugszeichenliste**

| | | | |
|---|---|---|---|
| 1 | Gehäuse | 6 | Faltenbalg |
| 1' | Frontgehäuseabschnitt | 6' | Adapterstück |
| 2 | Antriebsgehäuse | 7 | EDV-Vorrichtung |
| | | 70 | Akku-Einheit |
| 3 | Bohrfutter | 71 | Rastabschnitt |
| 30 | Aufnahmeelement | 72 | Luftfilter |
| 300 | Zylindrischer Abschnitt | | |
| 301 | Ausnehmung | 8 | Kabel |
| 302 | Zapfenabschnitt | 8' | Anschluss |
| 303 | Abflachung | 80 | Pneumatikschlauch |
| 304 | Innenwandsegmente | 81 | Gegenrastabschnitt |
| 305 | Anschlag | 82 | Kompressor |
| 31 | Klemmeinsatz | | |
| 310 | Gegenformkörper | 9 | Bedienelement |
| 311 | Anschlagscheibe | 90 | Anzeige |
| 312 | Durchtrittsöffnung | | |
| 32 | Klemmkörper | 10 | Bohrwiderstandsmessgerät |
| | | 11 | Griff |
| 4 | Bohrnadel | 12,12' | Teleskoprohrabschnitte |
| 4' | Abgeflachter Endabschnitt | 13 14 | Druckventil Befestigungsvorrichtung |
| 5 | Bohrnadelaustrittsführung | 15 | Andock-Flansch |
| 5' | Bohrnadelaustrittsöffnung | 15' | Andock-Teleskoprohrabschnitt |
| 51 | äußere Führungshülse | 16 | Führungsstange |
| 52 | Ringflansch | 17 | Antrieb/Motor |
| 53 | Schraubring | 18 | Lagerung |
| 54 | Teleskopaufnahmehülse | | |
| 55 | Innere Führungshülse | 20,21 | Gehäuseabschnitte |
| 56 | Überbrückungshülse | 22 | Motorgehäuse |
| 57 | Teleskopanschlag | | |
| 58 | Scheibe | | |

## Patentansprüche

1. Bohrwiderstandsmessgerät (10) zur Materialprüfung in feuchter Umgebung oder unter Wasser, wobei das Bohrwiderstandsmessgerät (10) ein Gehäuse (1) aufweist, in dem ein Antrieb und ein mit dem Antrieb gekoppeltes Bohrfutter (3) mit einer darin lösbar angeordneten Bohrnadel (4) angeordnet ist,
wobei das Gehäuse (1) eine von einer Bohrnadelsaustrittsführung (5) umfasste Bohrnadelaustrittsöffnung (5') hat, durch die sich die Bohrnadel (4) aus dem Gehäuse (1) erstreckt,
**dadurch gekennzeichnet, dass**
das Bohrwiderstandsmessgerät (10) zumindest einen wasserdichten Faltenbalg (6) aufweist, der im Inneren des Gehäuses (1) um die Bohrnadel (4) zwischen dem Bohrfutter (3) und der Bohrnadelsaustrittsführung (5) angeordnet ist, und wobei durch die Bohrnadelaustrittsöffnung (5') Feuchtigkeit oder Wasser in den Faltenbalg (6) eintreten kann, wobei der zumindest eine Faltenbalg (6) einenends um das Bohrfutter (3) an einem Antriebsgehäuse (2) und anderenends um die Bohrnadelaustrittsöffnung (5') an der Bohrnadelaustrittsführung (5) oder an dem Gehäuse (1) abdichtend befestigt ist, und wobei das Bohrfutter (3) und der Antrieb mittels einer Kupplungsvorrichtung verbunden sind, die eine kontaktfreie Drehmomentübertragung von dem Antrieb auf das Bohrfutter (3) bereitstellt.

2. Bohrwiderstandsmessgerät (10) nach Anspruch 1,
**dadurch gekennzeichnet, dass**
zwischen dem Bohrfutter (3) und der Bohrnadelaustrittsführung (5) zumindest zwei Faltenbalge (6) in Reihe aneinander angeordnet sind, die jeweils über ein Adapterstück (6') miteinander verbunden sind.

3. Bohrwiderstandsmessgerät (10) nach zumindest Anspruch 1 oder 2,
**dadurch gekennzeichnet, dass**
die Kupplungsvorrichtung eine Magnetkupplungsvorrichtung ist, bei der ein antriebsseitiger Magnetkupplungspartner mit einem bohrfutterseitigen Magnetkupplungspartner in Wirkverbindung steht, der eine hohlzylindrische Magnethalterung für ein Aufnahmeelement (30) des Bohrfutters (3) aufweist, das zur lösbaren Aufnahme eines abgeflachten Endabschnitts (4') der Bohrnadel (4) ausgebildet ist.

4. Bohrwiderstandsmessgerät (10) nach Anspruch 3,
**dadurch gekennzeichnet, dass**
das Aufnahmeelement (30) zumindest einen zylindrischen Abschnitt (300) mit einer Ausnehmung (301) aufweist, und das Bohrfutter (3) zumindest einen Klemmkörper (32) und einen Klemmeinsatz (31) aufweist, wobei
der Klemmeinsatz (31) Gegenformkörper (310) aufweist, der oder die den zumindest einen Klemmkörper (32) ergänzt/ergänzen, und der oder die sich von einer Anschlagscheibe (311) weg erstreckt/erstrecken, die an einer Stirnfläche des zylindrischen Abschnitts (300) anliegt und die eine Durchtrittsöffnung (312) für die Bohrnadel (4) aufweist,
wobei der oder die Gegenformkörper (310) und der zumindest eine Klemmkörper (32) in Abmessungen und Form zur Anordnung in der Ausnehmung (301) und zur klemmenden Aufnahme des abgeflachten Endabschnitts (4') der Bohrnadel (4) zwischen dem oder den Klemmkörper(n) (32) und den Gegenformkörpern (310) ausgebildet sind.

5. Bohrwiderstandsmessgerät (10) nach Anspruch 3 oder 4,
**dadurch gekennzeichnet, dass**
ein Ringmagnet die hohlzylindrische Magnethalterung bildet und beabstandet und kontaktfrei koaxial um den zylindrischen Abschnitt (300) des Aufnahmeelements (30) angeordnet ist, wobei bevorzugt ein magnetisch beeinflussbarer Distanzring den zylindrischen Abschnitt (300) umgibt.

6. Bohrwiderstandsmessgerät (10) nach zumindest einem der Ansprüche 1 bis 5,
**dadurch gekennzeichnet, dass**
das Bohrwiderstandsmessgerät (10) eine Ventilvorrichtung (13) aufweist, die
- zwischen dem Inneren des zumindest einen Faltenbalgs (6) und der Umgebung des Bohrwiderstandsmessgeräts (10) eine fluidische Verbindung bildet, bevorzugt an der Bohrnadelaustrittsführung (5) oder an dem Gehäuse (1) um die Bohrnadelaustrittsführung (5) vorgesehen ist, und
- in einer Richtung zum Wasseraustritt aus dem Inneren des zumindest einen Faltenbalgs (6) in die Umgebung bei Erreichen eines vorbestimmten Drucks im Inneren des zumindest einen Faltenbalgs (6) öffnet und in der entgegengesetzten Richtung zumindest bis zu einem zweiten vorbestimmten Druck im Inneren des zumindest einen Faltenbalgs (6) sperrt.

7. Bohrwiderstandsmessgerät (10) nach zumindest einem der Ansprüche 1 bis 6,
**dadurch gekennzeichnet, dass**
die Bohrnadel (4) in einer teleskopierbaren Führungsvorrichtung aus einer Mehrzahl von becherartigen Rohrabschnitten (12,12') unterschiedlichen Durchmessers geführt wird, wobei sich die teleskopierbare Führungsvorrichtung innerhalb des zumindest einen Faltenbalgs (6) zwischen dem Bohrfutter (3) und der Bohrnadelsaustrittsführung (5) um die Bohrnadel (4) erstreckt.

8. Bohrwiderstandsmessgerät (10) nach zumindest einem der Ansprüche 1 bis 7,
**dadurch gekennzeichnet, dass**
die Bohrnadelaustrittsführung (5) eine Führungshülse (55) mit einer Durchtrittsöffnung aufweist, in der die Bohrnadel (4) ohne Dichtmittel geführt wird, wobei die Durchtrittsöffnung der Führungshülse (55) so bemessen ist, dass ein Spalt zwischen der Bohrnadel (4) und der Führungshülse (55) eine Breite von höchstens 5%, vorzugsweise 1 bis 2 % des Bohrnadeldurchmessers aufweist.

9. Bohrwiderstandsmessgerät (10) nach zumindest einem der Ansprüche 1 bis 8,
**dadurch gekennzeichnet, dass**
das Bohrwiderstandsmessgerät (10) eine Akku-Einheit (70) zur Versorgung des Antriebs mit elektrischer Energie umfasst, wobei
- die Akku-Einheit (70) einen elektrischen Anschluss und einen Rastabschnitt (71) hat und wobei
- das Bohrwiderstandsmessgerät (10) einen korrespondierenden elektrischen Anschluss und einen korrespondierenden Gegenrastabschnitt (81) aufweist, wobei
- in einer Eingriffsanordnung der Rastabschnitt (71) der Akku-Einheit (70) mit dem Gegenrastabschnitt (81) des Bohrwiderstandsmessgeräts (10) in Eingriff steht, und die elektrischen Anschlüsse der Akku-Einheit (70) und des Bohrwiderstandsmessgeräts (10) in Kontakt stehen,
- in einer entkoppelten Anordnung der Rastabschnitt (71) der Akku-Einheit (70) von dem Gegenrastabschnitt (81) des Bohrwiderstandsmessgeräts (10) gelöst ist und der elektrische Anschluss der Akku-Einheit (70) durch ein Kabel (8) mit dem elektrischen Anschluss des Bohrwiderstandsmessgeräts (10) verbunden ist.

10. Bohrwiderstandsmessgerät (10) nach zumindest einem der Ansprüche 1 bis 9,
**dadurch gekennzeichnet, dass**
das Bohrwiderstandsmessgerät (10) im Antriebsabschnitt einen Kompressor (82) zur Bereitstellung eines Überdrucks in dem Gehäuse (1) aufweist, der mit einem Luftfilter (72) verbunden ist,
wobei der Luftfilter (72) bevorzugt an der Akku-Einheit (70) angeordnet ist, wobei die Akku-Einheit (70) und das Gehäuse (1) Pneumatikanschlüsse aufweisen, die in der Eingriffsanordnung der Akku-Einheit (70) miteinander oder in der entkoppelten Anordnung der Akku-Einheit (70) über einen Schlauch (80) miteinander verbunden sind.

11. Verfahren zur Bohrwiderstandsmessung mit einem Bohrwiderstandsmessgerät nach zumindest einem der Ansprüche 1 bis 10,
umfassend die Schritte:
- Ansetzen des Bohrwiderstandsmessgeräts (10) in feuchter Umgebung oder unter Wasser an ein zu prüfendes Objekt (20), dabei
- eindringen Lassen von Feuchtigkeit oder Wasser durch die Bohrnadelaustrittsöffnung (5') in den zumindest einen Faltenbalg (6);
- Vorschieben der Bohrnadel (4) und Einführen in das zu prüfende Objekt (20), dabei Zusammenschieben des zumindest einen Faltenbalgs (6) und bei Erreichen eines vorbestimmten Drucks innerhalb des zusammengeschobenen Faltenbalgs (6) austreten Lassen der Feuchtigkeit oder des Wassers in die Umgebung des Bohrwiderstandsmessgeräts (10), vorzugsweise durch Öffnen einer mit dem Inneren des zumindest einen Faltenbalgs (6) und der Umgebung in Verbindung stehenden Ventilvorrichtung (13).

## Claims

1. A drilling-resistance measuring device (10) for testing of materials in a moist environment or for use underwater, wherein the drilling-resistance measuring device (10) has a housing (1) in which are arranged a drive and a drill chuck (3) that is coupled to the drive and has a drill needle arranged therein in a detachable manner,
wherein the housing (1) has a drill needle exit opening (5') enclosed by a drill needle exit guide (5), with the drill needle (4) extending out of the housing (1) through said drill needle exit opening (5'),
**characterised in that**
the drilling-resistance measuring device (10) has at least one watertight bellows (6) which is arranged in the interior of the housing (1) around the drill needle (4) between the drill chuck (3) and the drill needle exit guide (5), and wherein moisture or water may enter into the bellows (6) through the drill needle exit opening (5'), wherein, at one end, the at least one bellows (6) is sealingly fastened to a drive housing (2) around the drill chuck (3) and, at the other end, to the drill needle exit guide (5) or to the housing (1) around the drill needle exit opening (5'), and wherein the drill chuck (3) and the drive are connected by means of a coupling device which provides non-contact torque transmission from the drive to the drill chuck (3).

2. The drilling-resistance measuring device (10) in accordance with Claim 1,
**characterised in that**
at least two bellows (6) are arranged in series connection between the drill chuck (3) and the drill needle exit guide (5), said at least two bellows (6) being connected to each other via an adapter piece (6').

3. The drilling-resistance measuring device (10) in accordance with Claim 1 or 2,
**characterised in that**
the coupling device is a magnetic coupling device in which a magnetic coupling partner on the drive side is operatively connected to a magnetic coupling partner on the drill chuck side, said magnetic coupling partner on the drill chuck side having a hollow cylindrical magnetic holder for a receiving element (30) of the drill chuck (3), said receiving element (30) being configured to receive a flattened end section (4') of the drill needle (4) in a detachable manner.

4. The drilling-resistance measuring device (10) in accordance with Claim 3,
**characterised in that**
the receiving element (30) has at least one cylindrical section (300) with a recess (301) and the drill chuck (3) has at least one clamping body (32) and one clamping insert (31), wherein
the clamping insert (31) has a mating moulded body (310) or mating moulded bodies (310) which supplements/supplement the at least one clamping body (32) and which extends/extend away from a stop washer (311) that abuts against a front face of the cylindrical section (300) and has a through opening (312) for the drill needle (4),
wherein, in their dimensions and shape, the mating moulded body (310) or the mating moulded bodies (310) and the at least one clamping body (32) are configured to be arranged in the recess (301) and to clampingly receive the flattened end section (4') of the drill needle (4) between the clamping body (32) or the clamping bodies (32) and the mating moulded bodies (310).

5. The drilling-resistance measuring device (10) in accordance with Claim 3 or 4,
**characterised in that**
a ring magnet forms the hollow cylindrical magnetic holder and is coaxially arranged around the cylindrical section (300) of the receiving element (30) in a spaced and non-contact manner, wherein a spacer ring capable of being magnetically influenced preferably surrounds the cylindrical section (300).

6. The drilling-resistance measuring device (10) in accordance with at least any one of Claims 1 to 5,
**characterised in that**
the drilling-resistance measuring device (10) has a valve device (13) which
- forms a fluid communication between the interior of the at least one bellows (6) and the environment of the drilling-resistance measuring device (10), preferably is provided at the drill needle exit guide (5) or at the housing (1) around the drill needle exit guide (5), and
- opens in one direction towards the exit of water from the interior of the at least one bellows (6) into the environment when a predefined pressure is reached in the interior of the at least one bellows (6) and blocks in the opposite direction at least until a second predefined pressure is reached in the interior of the at least one bellows (6).

7. The drilling-resistance measuring device (10) in accordance with at least any one of Claims 1 to 6,
**characterised in that**
the drill needle (4) is guided in a telescopic guiding device from a plurality of cup-like pipe sections (12, 12') having different diameters, wherein the telescopic guiding device extends inside the at least one bellows (6) between the drill chuck (3) and the drill needle exit guide (6) around the drill needle (4).

8. The drilling-resistance measuring device (10) in accordance with at least any one of Claims 1 to 7,
**characterised in that**
the drill needle exit guide (5) has a guide sleeve (55) with a through opening in which the drill needle (4) is guided without any sealing means, wherein the through opening of the guide sleeve (55) is dimensioned such that a gap between the drill needle (4) and the guide sleeve (55) has width of no more than 5 %, preferably 1 to 2 % of the drill needle diameter.

9. The drilling-resistance measuring device (10) in accordance with at least any one of Claims 1 to 8,
**characterised in that**
the drilling-resistance measuring device (10) comprises a battery unit (70) for supplying the drive with electrical energy, wherein
- the battery unit (70) has an electric connector and a detent section (71), and wherein
- the drilling-resistance measuring device (10) has a corresponding electric connector and a corresponding detent counter-section (81), wherein
- in an engagement arrangement, the detent section (71) of the battery unit (70) engages the detent counter-section (81) of the drilling-resistance measuring device (10) and the electric connectors of the battery unit (70) and the drilling-resistance measuring device (10) contact each other,
- in a disengagement arrangement, the detent section (71) of the battery unit (70) is disengaged from the detent counter-section (81) of the drilling-resistance measuring device (10) and the electric connector of the battery unit (70) is connected to the electric connector of the drilling-resistance measuring device (10) via cable (8).

10. The drilling-resistance measuring device (10) in accordance with at least any one of Claims 1 to 9,
**characterised in that**
the drilling-resistance measuring device (10) has a compressor (82) in the drive section for providing an excess pressure in the housing (1), said compressor (82) being connected to an air filter (72),
wherein the air filter (72) is preferably arranged on the battery unit (70), wherein the battery unit (70) and the housing (1) have pneumatic connectors which, in the engagement arrangement of the battery unit (70), are connected to each other or, in the disengagement arrangement of the battery unit (70), are connected to each other via tube (80).

11. A method for drilling resistance measurement using a drilling-resistance measuring device in accordance with at least any one of Claims 1 to 10, comprising the steps of:
- applying the drilling-resistance measuring device (10) to an object (20) to be tested in a moist environment or underwater, therein
- letting moisture or water enter into the at least bellows (6) through the drill needle exit opening (5'),
- advancing the drill needle (4) and inserting into the object (20) to be tested, therein collapsing the at least one bellows (6) and, when a predefined pressure is reached inside the collapsed bellows (6), letting the moisture or the water exit into the environment of the drilling-resistance measuring device (10), preferably by opening a valve device (13) being in connection with the interior of the at least one bellows (6) and the environment.

## Revendications

1. Appareil de mesure de résistance au forage (10) pour le contrôle de matériau dans un environnement humide ou sous l'eau, dans lequel l'appareil de mesure de résistance au forage (10) présente un logement (1) dans lequel un entraînement et un mandrin de forage (3) couplé à l'entraînement avec une aiguille de forage (4) disposée de manière amovible en son sein sont disposés,
dans lequel le logement (1) a une ouverture de sortie d'aiguille de forage (5') comprise par un guide de sortie d'aiguille de forage (5) à travers laquelle l'aiguille de forage (4) s'étend hors du logement (1),
**caractérisé en ce que**
l'appareil de mesure de résistance au forage (10) présente au moins un soufflet étanche à l'eau (6) qui est disposé à l'intérieur du logement (1) autour de l'aiguille de forage (4) entre le mandrin de forage (3) et le guide de sortie d'aiguille de forage (5), et dans lequel de l'humidité ou de l'eau peut pénétrer dans le soufflet (6) à travers l'ouverture de sortie d'aiguille de forage (5'), dans lequel l'au moins un soufflet (6) est fixé de manière étanche à une extrémité autour du mandrin de forage (3) à un logement d'entraînement (2) et à l'autre extrémité autour de l'ouverture de sortie d'aiguille de forage (5') au guide de sortie d'aiguille de forage (5) ou au logement (1), et dans lequel le mandrin de forage (3) et l'entraînement sont connectés au moyen d'un dispositif d'accouplement qui fournit une transmission de couple de rotation sans contact de l'entraînement au mandrin de forage (3).

2. Appareil de mesure de résistance au forage (10) selon la revendication 1,
**caractérisé en ce que**
au moins deux soufflets (6) sont disposés en rang l'un contre l'autre entre le mandrin de forage (3) et le guide de sortie d'aiguille de forage (5), lesquels sont chacun connectés l'un à l'autre par le biais d'un raccord (6').

3. Appareil de mesure de résistance au forage (10) selon au moins la revendication 1 ou 2,
**caractérisé en ce que**
le dispositif d'accouplement est un dispositif d'accouplement magnétique pour lequel un partenaire d'accouplement magnétique côté entraînement est en connexion active avec un partenaire d'accouplement magnétique côté mandrin de forage qui présente un support magnétique cylindrique creux pour un élément de réception (30) du mandrin de forage (3) qui est réalisé pour la réception amovible d'une section d'extrémité aplatie (4') de l'aiguille de forage (4).

4. Appareil de mesure de résistance au forage (10) selon la revendication 3,
**caractérisé en ce que**
l'élément de réception (30) présente au moins une section cylindrique (300) avec un évidement (301) et le mandrin de forage (3) présente au moins un corps de serrage (32) et un insert de serrage (31), dans lequel l'insert de serrage (31) présente des corps de moule conjugués (310) qui complète(nt) l'au moins un corps de serrage (32) et qui s'étend(ent) à l'écart d'un disque de butée (311) qui repose sur une face frontale de la section cylindrique (300) et qui présente une ouverture de passage (312) pour l'aiguille de forage (4),
dans lequel le ou les corps de moule conjugué(s) (310) et l'au moins un corps de serrage (32) sont réalisés dans des dimensions et une forme pour l'agencement dans l'évidement (301) et pour la réception par serrage de la section d'extrémité aplatie (4') de l'aiguille de forage (4) entre le ou les corps de serrage (32) et les corps de moule conjugués (310).

5. Appareil de mesure de résistance au forage (10) selon la revendication 3 ou 4,
**caractérisé en ce que**
un aimant torique forme le support magnétique cylindrique creux et est disposé de manière écartée et sans contact coaxialement autour de la section cylindrique (300) de l'élément de réception (30), dans lequel une bague d'écartement pouvant être influencée magnétiquement entoure de préférence la section cylindrique (300).

6. Appareil de mesure de résistance au forage (10) selon au moins une des revendications 1 à 5,
**caractérisé en ce que**
l'appareil de mesure de résistance au forage (10) présente un dispositif de soupape (13) qui
- forme une connexion fluidique entre l'intérieur de l'au moins un soufflet (6) et l'environnement de l'appareil de mesure de résistance au forage (10), est de préférence prévu sur le guide de sortie d'aiguille de forage (5) ou sur le logement (1) autour du guide de sortie d'aiguille de forage (5), et
- s'ouvre dans une direction par rapport à la sortie d'eau de l'intérieur de l'au moins un soufflet (6) dans l'environnement lors de l'atteinte d'une pression prédéterminée à l'intérieur de l'au moins un soufflet (6) et se ferme dans la direction opposée au moins jusqu'à une seconde pression prédéterminée à l'intérieur de l'au moins un soufflet (6).

7. Appareil de mesure de résistance au forage (10) selon au moins une des revendications 1 à 6,
**caractérisé en ce que**
l'aiguille de forage (4) est guidée dans un dispositif de guidage pouvant être télescopé constitué d'une pluralité de sections de tube de type godet (12, 12') de diamètre différent, dans lequel le dispositif de guidage pouvant être télescopé s'étend au sein de l'au moins un soufflet (6) entre le mandrin de forage (3) et le guide de sortie d'aiguille de forage (5) autour de l'aiguille de forage (4).

8. Appareil de mesure de résistance au forage (10) selon au moins une des revendications 1 à 7,
**caractérisé en ce que**
le guide de sortie d'aiguille de forage (5) présente un manchon de guidage (55) avec une ouverture de passage dans lequel l'aiguille de forage (4) est guidée sans moyen d'étanchéité, dans lequel l'ouverture de passage du manchon de guidage (55) est mesurée de sorte qu'un interstice entre l'aiguille de forage (4) et le manchon de guidage (55) présente une largeur de 5 % maximum, de préférence 1 à 2 % du diamètre de l'aiguille de forage.

9. Appareil de mesure de résistance au forage (10) selon au moins une des revendications 1 à 8,
**caractérisé en ce que**
l'appareil de mesure de résistance au forage (10) comprend un module d'accumulateur (70) pour l'alimentation de l'entraînement en énergie électrique, dans lequel
- le module d'accumulateur (70) a un raccord électrique et une section d'encliquetage (71), et dans lequel
- l'appareil de mesure de résistance au forage (10) présente un raccord électrique correspondant et une section d'encliquetage conjuguée correspondante (81), dans lequel
- la section d'encliquetage (71) du module d'accumulateur (70) s'engrène avec la section d'encliquetage conjuguée (81) de l'appareil de mesure de résistance au forage (10) dans un agencement d'engrènement, et les raccords électriques (81) du module d'accumulateur (70) et de l'appareil de mesure de résistance au forage (10) sont en contact,
- la section d'encliquetage (71) du module d'accumulateur (70) est détachée de la section d'encliquetage conjuguée (81) de l'appareil de mesure de résistance au forage (10) dans un agencement découplé, et le raccord électrique du module d'accumulateur (70) est connecté par un câble (8) au raccord électrique de l'appareil de mesure de résistance au forage (10).

10. Appareil de mesure de résistance au forage (10) selon au moins une des revendications 1 à 9,
**caractérisé en ce que**
l'appareil de mesure de résistance au forage (10) présente dans la section d'entraînement un compresseur (82) pour la fourniture d'une surpression dans le logement (1), lequel est connecté à un filtre à air (72),
dans lequel le filtre à air (72) est de préférence disposé sur le module d'accumulateur (70), dans lequel le module d'accumulateur (70) et le logement (1) présentent des raccords pneumatiques qui sont connectés l'un à l'autre dans l'agencement d'engrènement du module d'accumulateur (70) ou l'un à l'autre par le biais d'un tuyau (80) dans l'agencement découplé du module d'accumulateur (70).

11. Procédé de mesure de résistance au forage avec un appareil de mesure de résistance au forage selon au moins une des revendications 1 à 10, comprenant les étapes consistant à :
- fixer l'appareil de mesure de résistance au forage (10) dans un environnement humide ou sous l'eau sur un objet à contrôler (20), ce faisant
- laisser pénétrer de l'humidité ou de l'eau à travers l'ouverture de sortie d'aiguille de forage (5') dans l'au moins un soufflet (6) ;
- avancer l'aiguille de forage (4) et l'introduire dans l'objet à contrôler (20), regrouper ce faisant l'au moins un soufflet (6) et lors de l'atteinte d'une pression prédéterminée au sein du soufflet regroupé (6), laisser sortir l'humidité ou l'eau dans l'environnement de l'appareil de mesure de résistance au forage (10), de préférence par ouverture d'un dispositif de soupape (13) en connexion avec l'intérieur de l'au moins un soufflet (6) et l'environnement.
